(19) **Europäisches Patentamt**
**European Patent Office**
**Office européen des brevets**

(11) **EP 2 179 989 A2**

(12) **EUROPÄISCHE PATENTANMELDUNG**

(43) Veröffentlichungstag:
28.04.2010   Patentblatt 2010/17

(51) Int Cl.:
*C07D 213/69* (2006.01)   *C07D 213/643* (2006.01)
*A61K 31/4409* (2006.01)

(21) Anmeldenummer: 09172981.4

(22) Anmeldetag: 14.10.2009

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**
Benannte Erstreckungsstaaten:
**AL BA RS**

(30) Priorität: **25.10.2008   DE 102008053242**

(71) Anmelder: **Saltigo GmbH**
**40764 Langenfeld (DE)**

(72) Erfinder:
• **Kreis, Michael**
**51373, Leverkusen (DE)**
• **Beckmann, Edith**
**50735, Köln (DE)**

(74) Vertreter: **Siegers, Britta**
**LANXESS Deutschland GmbH**
**51369 Leverkusen (DE)**

(54) **Verfahren zur Herstellung von Heteroaryl-Arylethers**

(57)    Die vorliegende Erfindung betrifft ein Verfahren zur Herstellung von gegebenenfalls substituierten Heteroaryl-Arylethern, insbesondere von Phenoxypyridinen.

**Beschreibung**

Die vorliegende Erfindung betrifft ein Verfahren zur Herstellung von gegebenenfalls substituierten Heteroaryl-Arylethem, insbesondere von Phenoxypyridinen.

Heteroaryl-Arylether sind von großer Bedeutung als Feinchemikalien und Zwischenprodukte für die Herstellung von Arzneimitteln, wie z.B. Antidepressiva, Antibiotika oder Serotoninwiederauf nahmehemmern und Agrochemikalien.

Es sind verschiedene Verfahren zur Herstellung von Heteroaryl-Arylethem bekannt.

Ihre Darstellung kann beispielsweise über die Reaktion von Pyridylpyridiniumsalzen mit Phenolen unter Verwendung von Basen erfolgen (Chem. Ber. (1956), 89, 2921-2933; JP 2001002644 A). Nachteilig an diesem Verfahren ist, dass dabei Pyridilpyridiniumsalze zunächst aus den entsprechenden chlorierten Pyridinen kondensiert werden müssen und anschließend Pyridin als nichtverwendbares Nebenprodukt anfällt. Daher ist dieses Verfahren ökologisch und ökonomisch nachteilig.

Chemg et al. (Tetrahedron 58 (2002), 4931-4935) beschreiben die Herstellung von Heteroaryl-Arylethem durch Substitution von Halogenpyridinen mit Nukleophilen in polaren Lösungsmitteln unter Mikrowellenbestrahlung. Nachteilig bei diesem Verfahren ist, dass zum Erhalt guter Ausbeuten teure Edukte, wie z.B. 4-Iodpyridin, eingesetzt werden müssen und lediglich schlechte Ausbeuten erhalten werden.

Angelo et al. (Tetrahedron Letters 47 (2006) 5045-5048) beschreiben die Umsetzung von Chlorheterozyklen mit Phenolderivaten unter Mikrowellenbestrahlung in Gegenwart von Kupferpulver als Katalysator und Cäsiumcarbonat als Base zur Herstellung von substituierten Heteroaryl-Arylethem. Aufgrund der Toxizität des Kupfers, sowie der Verwendung einer Mikrowellenheizung und des teuren Cäsiumcarbonats, ist das Verfahren für den technischen Einsatz nicht geeignet.

In DE 69829048 T2 wird ein Verfahren beschrieben, das durch Substitution von 4-Chlorpyridinhydrochlorid mit 4-Hydroxybenzaldehyd unter Verwendung von Kaliumcarbonat als Base in N,N-Dimethylformamid zu 4-(4-Pyridinoxy)benzaldehyd führt. Nachteilig bei diesem Verfahren ist, dass lediglich geringe Ausbeuten von maximal 13 % d. Th. erhalten werden.

In DE 60201819 T2 wird die Herstellung von Heteroaryl-Arylethem durch Substitution von Pyridinylen durch Phenolate unter milden Bedingungen beschrieben. Nachteilig bei diesem Verfahren ist ebenfalls die geringe Ausbeute und die Limitierung der Edukte auf mit elektronenschiebenden Resten substituierten Pyridinylen.

Den vorstehenden Verfahren ist gemeinsam, dass sie entweder keine guten Ausbeuten liefern oder teure Spezialchemikalien erfordern und daher für die technische Produktion von Heteroaryl-Arylethern ungeeignet sind. Es bestand folglich das Bedürfnis ein Verfahren bereitzustellen, dass sich für die effiziente Herstellung von Heteroaryl-Arylethern eignet.

Es wurde nun überraschend gefunden, dass die Umsetzung eines gegebenenfalls substituierten Phenols mit einem, gegebenenfalls substituierten, Heteroaryl-Halogenid oder Heteroaryl-Pseudohalogeniden, unter geeigneten Reaktionsbedingungen unter hohen chemischen Ausbeuten zu einem Heteroaryl-Arylether abläuft.

Gegenstand der Erfindung ist daher ein Verfahren zur Herstellung von Verbindungen der Formel (I)

$$\text{ARYL-O-HETEROARYL} \qquad \text{(I)}$$

wobei ARYL für $C_6\text{-}C_{20}$-Aryl steht, das gegebenenfalls einfach oder mehrfach mit Resten substituiert ist, die unabhängig voneinander ausgewählt sind aus der Gruppe

Alkyl, Alkenyl, Alkoxy, Alkoxycarbonyl, Alkoxycarbonylamino, Dialkylamino, Aryl, Arylalkyl, Halogen, Halogenalkyl, Halogenalkylen, Halogenalkoxy, Halogenalkylthio, 5- bis 6-gliedriges Heteroaryl, und 3- bis 7-, gliedriger gesättigter oder teilweise ungesättigter Heterozyklus

und HETEROARYL für Pyrazinyl, Pyridyl, Pyrimidinyl oder Pyridazinyl steht, das gegebenenfalls einfach oder mehrfach mit Resten substituiert ist, die unabhängig voneinander ausgewählt sind aus der Gruppe

Alkyl, Alkenyl, Alkoxy, Alkoxycarbonyl, Alkoxycarbonylamino, Dialkylamino, Aryl, Arylalkyl, Halogen, Halogenalkyl, Halogenalkylen, Halogenalkoxy, Halogenalkylthio, 5- bis 6-gliedriges Heteroaryl, 3- bis 7- gliedriger gesättigter oder teilweise ungesättigter Heterozyklus

das dadurch gekennzeichnet ist, dass Verbindungen der Formel (II)

$$\text{ARYL-O}^-\ \text{Kat}^+ \qquad \text{(II)}$$

wobei ARYL die vorgenannte Bedeutung besitzt und $\text{Kat}^+$ ein beliebiges einfach geladenes Kation oder ein $1/n$-tes Äquivalent eines $n$-wertigen Kations darstellt, mit einer Verbindung der Formel (III) umgesetzt wird

$$\text{HETEROARYL-Y} \qquad \text{(III)}$$

wobei HETEROARYL die vorgenannte Bedeutung besitzt und Y für ein Halogen oder Pseudohalogen steht.

Der Rahmen der Erfindung umfasst alle oben stehenden und im Folgenden aufgeführten, allgemeinen oder in Vorzugsbereichen genannten Restedefinitionen, Parameter und Erläuterungen untereinander, also auch zwischen den jeweiligen Bereichen und Vorzugsbereichen in beliebiger Kombination.

**Alkyl bzw. Alkenyl bzw. Alkoxy** steht im Rahmen der Erfindung für einen geradkettigen, zyklischen, verzweigten oder unverzweigten Alkyl- bzw. Alkenyl- bzw. Alkoxy- Rest mit 1 bis 15 bzw. 2 bis 6 bzw. mit 1 bis 6 Kohlenstoffatomen.

Beispielhaft und vorzugsweise steht Alkyl für Methyl, Ethyl, n-Propyl, Isopropyl, n-, i-, s- oder t-Butyl, Cyclopropyl, Cyclobutyl, Cyclopentyl, Cyclohexyl, Cycloheptyl, n-Pentyl, 1-Methylbutyl, 2-Methylbutyl, 3-Methylbutyl, neo-Pentyl, 1-Ethylpropyl, cyclo-Hexyl, cyclo-Pentyl, n-Hexyl, n-Heptyl, n-Octyl, n-Decyl und n-Dodecyl

Beispielhaft und vorzugsweise steht Alkenyl für Vinyl, Allyl, Isopropenyl und n-But-2-en-1-yl.

Beispielhaft und vorzugsweise steht Alkoxy für Methoxy, Ethoxy, n-Propoxy, Isopropoxy, t-Butoxy, n-Pentoxy und n-Hexoxy.

**Alkoxycarbonyl** steht im Rahmen der Erfindung vorzugsweise für einen geradkettigen oder verzweigten Alkoxyrest mit 1 bis 6 Kohlenstoffatomen, der über eine Carbonylgruppe verknüpft ist. Beispielhaft und vorzugsweise seien genannt: Methoxycarbonyl, Ethoxycarbonyl, n-Propoxycarbonyl, Isopropoxycarbonyl und t-Butoxycarbonyl.

**Alkoxycarbonylamino** steht im Rahmen der Erfindung für eine Amino-Gruppe mit einem geradkettigen oder verzweigten Alkoxycarbonylsubstituenten, der vorzugs-weise im Alkoxyrest 1 bis 6 Kohlenstoffatome aufweist und über die Carbonylgruppe verknüpft ist. Beispielhaft und vorzugsweise seien genannt: Methoxycarbonyl-amino, Ethoxycarbonylamino, n-Propoxycarbonylamino und t-Butoxycarbonylamino.

**Aryl** steht im Rahmen der Erfindung für einen mono-, bi- oder trizyklischen carbozyklischen aromatischen Rest mit vorzugsweise 6 bis 20 aromatischen Kohlenstoffatomen ($C_6$-$C_{20}$-Aryl). Weiterhin können die carbozyklischen aromatischen Reste mit bis zu fünf gleichen oder verschiedenen Substituenten pro Zyklus substituiert sein, ausgewählt aus der Gruppe Alkyl, Alkenyl, Alkoxy, Alkoxycarbonyl, Alkoxycarbonylamino, Aryl, Arylalkyl, Dialkylamino, Halogen, Halogenalkyl, Halogenalkylen, Halogenalkoxy, Halogenalkylthio, 5- bis 6-gliedriges Heteroaryl und 3- bis 7-gliedriger gesättigter oder teilweise ungesättigter Heterozyklus. Beispielhaft und bevorzugt steht $C_6$-$C_{20}$-Aryl für Biphenyl, Phenyl, Naphthyl, Phenanthrenyl, Anthracenyl oder Fluorenyl.

**Arylalkyl** bedeutet jeweils unabhängig voneinander einen geradkettigen, zyklischen, verzweigten oder unverzweigten Alky-Rest nach vorstehender Definition, der einfach, mehrfach oder vollständig durch Aryl-Reste gemäß vorstehender Definition substituiert sein kann. Ein Beispiel für Arylalkyl ist Benzyl.

**Beispielhaft** und bevorzugt sind als **Halogene** Fluor, Chlor oder Brom, besonders bevorzugt ist Chlor.

**Dialkylamino** bzw. steht im Rahmen der Erfindung für eine Amino-Gruppe mit einem oder zwei gleichen oder verschiedenen, zyklischen, geradkettigen oder verzweigten Alkylsubstituenten, die vorzugsweise jeweils 1 bis 6 Kohlenstoffatomen aufweisen.

Beispielhaft und vorzugsweise steht Dialkylamino für *N,N*-Dimethylamino, *N,N*-Diethylamino, *N*-Ethyl-*N*-methylamino, *N*-Methyl-*N*-n-propylamino, *N*-Isopropyl-*N*-n-propylamino, *N*-t-Butyl-*N*-methylamino, *N*-Ethyl-*N*-n-pentylamino und *N*-n-Hexyl-*N*-methylamino.

**Halogenalkyl bzw. Halogenalkylen bzw. Halogenalkoxy** steht im Rahmen der Erfindung für einen geradkettigen, zyklischen, verzweigten oder unverzweigten Alkyl-, bzw. Alkylen-, bzw. Alkoxy-Rest nach vorstehender Definition, der einfach, mehrfach oder vollständig mit Halogenatomen substituiert ist.

Beispielhaft und vorzugsweise steht Halogenalkyl für Dichlormethyl, Difluormethyl, Fluormethyl, Trifluormethyl, Trichlormethyl, 2,2,2-Trifluorethyl, Pentafluorethyl, Heptafluorisopropyl und Nonafluorbutyl.

Beispielhaft und vorzugsweise steht Halogenalkylen für Chlorehtylen, Dichlorethylen oder Trifluorethylen.

Beispielhaft und vorzugsweise steht Halogenalkoxy für Difluormethoxy, Fluorethoxy, Fluormethoxy, Trifluormethoxy, Trichlormethoxy und 2,2,2-Trifluorethoxy.

**Halogenalkylthio** steht im Rahmen der Erfindung für einen geradkettigen, cyklischen, verzweigten oder unverzweigten Rest mit 1 bis 15 Kohlenstoffatomen, der einfach, mehrfach oder vollständig mit Halogenatomen substituiert ist. Beispielhaft und vorzugsweise steht Halogenalkylthio für Chlorethylthio, Chlorbutylthio, Chlorhexylthio, Chlorpentylthio, Chlordodecylthio, Dichlorethylthio, Fluorethylthio, Trifluormethylthio und 2,2,2-Trifluorethylthio.

**5- bis 6-gliedriges Heteroaryl** steht im Rahmen der Erfindung vorzugsweise für einen aromatischen Heterozyklus, mit bis zu 3 gleichen oder verschiedenen Heteroatomen aus der Reihe S, N und/oder 0, der über ein Ringkohlenstoffatom des Heteroaromaten, gegebenenfalls auch über ein Ringstickstoffatom des Heteroaromaten verknüpft ist. Beispielhaft seien genannt: Furanyl, Pyrrolyl, Thienyl, Thiazolyl, Oxazolyl, Imidazolyl, Triazolyl, Pyridyl, Pyrimidinyl, Pyridazinyl. Bevorzugt sind Pyridyl, Pyrimidinyl, Pyridazinyl, Furyl und Thiazolyl.

**3- bis 7-gliedriger gesättigter oder teilweise ungesättigter Heterozyklus** steht im Rahmen der Erfindung vorzugsweise für einen Heterozyklus, mit bis zu 3 gleichen oder verschiedenen Heteroatomen aus der Reihe S, N und/oder O, der über ein Ringkohlenstoffatom oder ein Ringstickstoffatom verknüpft ist und der eine oder zwei Doppelbindungen enthalten kann. Bevorzugt ist ein 5- bis 7-gliedriger gesättigter Heterozyklus mit bis zu 2 gleichen oder verschiedenen Heteroatomen aus der Reihe S, N und/oder O. Beispielhaft seien genannt: Tetrahydrofur-2-yl, Tetrahydrofur-3-yl, Pyrrolidin-1-yl, Pyrrolidin-2-yl, Pyrrolidin-3-yl, Pyrrolin-1-yl, Piperidin-1-yl, Piperidin-4-yl, 1,2-Dihydropyridin-1-yl, 1,4-Dihydropyridin-1-yl, Piperazin-1-yl, Morpholin-4-yl, Thiomorpholin-4-yl, Azepin-1-yl, 1,4-Diazepin-1-yl. Bevorzugt sind Piperidinyl, Piperazinyl, Morpholinyl und Pyrrolidinyl.

**Pseudohalogen** bezeichnet im Rahmen der Erfindung Reste, die in ihren chemischen Eigenschaften eine große Ähnlichkeit zu den Halogenen aufweisen. Dies sind z.B. Sulfonate und Halogensulfonate, wie z.B. Tosylat, Triflat, Mesylat und Nonafluorbutylsulfonat, aber auch Thiocyanat und Azid.

Bevorzugt steht ARYL für einen $C_6$-$C_{20}$-Arylrest, der gegebenenfalls einfach oder mehrfach mit Resten substituiert ist, die unabhängig voneinander ausgewählt sind aus der Gruppe Alkoxy, Dialkylamino, Halogenalkyl, Halogenalkylthio oder Halogenalkyloxy.

In einer besonders bevorzugten Ausführungsform steht ARYL für einen $C_6$-$C_{20}$-Arylrest, der gegebenenfalls einfach oder mehrfach mit Resten substituiert ist, die unabhängig voneinander ausgewählt sind aus der Gruppe Trifluormethoxy, Methoxy und Methyl. In einer ganz besonders bevorzugten Ausführungsform steht ARYL für einen $C_6$-$C_{20}$-Arylrest der gegebenenfalls einfach oder mehrfach durch Reste aus der Gruppe Trifluormethoxy, Methoxy und Methyl an der 2-,3- oder/und 4-Position substituiert ist.

In einer bevorzugten Ausführungsform steht HETEROARYL für Pyridyl der gegebenenfalls einfach oder mehrfach mit Resten substituiert ist, die unabhängig voneinander ausgewählt sind aus der Gruppe Alkoxy, Alkyl, Aryl, Halogenalkyl, Halogenalkylthio oder Halogenalkyloxy. In einer ganz besonders bevorzugten Ausführungsform steht HETEROARYL für Pyridyl das gegebenenfalls einfach oder mehrfach durch Reste aus der Gruppe Trifluormethoxy, Methoxy und Methyl an der 2- , 3- oder/und 4- Position substituiert ist.

Bevorzugte Verbindungen der Formel (II) sind Phenol, 4-Methoxyphenol, 2-Trifluormethoxyphenol, 4-Trifluormethoxyphenol und p-Kresol. Bevorzugte Verbindung der Formel (III) ist 4-Chlorpyridin. In einer weiteren besonders bevorzugten Ausführungsform sind die Verbindungen der Formel (I) 4-[4-Trifluormethoxyphenoxy]pyridin, 4-[4-Methylphenoxy]pyridin, 4-Phenoxypyridin, 4-[4-Methoxyphenoxy]pyridin und 4-[2-Trifluormethoxyphenoxy]pyridin.

Die Verbindungen der Formel (III) können beispielsweise durch Umsetzung von Verbindungen der Formel (IIIa)

$$\text{HETEROARYL-Y} * \text{HX} \qquad \text{(IIIa)}$$

mit einer Base hergestellt werden. HX steht für eine Protonensäure. Beispielhaft und vorzugsweise steht die Verbindung (IIIa) für die Salze der Verbindung (III) in Form des Hydrochlorids, Hydrobromids, Hydroiodids, Hydrogensulfats oder Hydrofluorids.

Die Verbindungen der Formel (II) können im Rahmen des erfindungsgemäßen Verfahrens beispielsweise durch Umsetzung von Verbindungen der Formel (IIa)

$$\text{ARYL-OH} \qquad \text{(IIa)}$$

mit einer geeigneten Base, die in der Lage ist die eingesetzten Phenole zu deprotonieren, hergestellt werden. Bevorzugt weisen die korrespondierende Säuren dieser Basen einen pKa-Wert > 10, gemessen unter Standardbedingungen, auf. Besonders bevorzugt ist der pKa-Wert > 15.

Als Beispiele für geeignete Basen seien hier aufgeführt Erdalkali- oder Alkalimetallhydride, - hydroxide, -amide, -alkoholate, oder -carbonate wie beispielsweise Natriumhydrid, Natriumamid, Lithiumdiethylamid, Natriummethylat, Kalium-tert.-butylat, Natriumhydroxid, Kaliumhydroxid, Natriumcarbonat, Kaliumcarbonat, sowie tertiäre Amine, wie Trimethylamin, Triethylamin, Tributylamin, Di-isopropyl-ethylamin, N,N-Dimethylanilin, Piperidin, N-Methylpiperidin, N,N-Dimethylaminopyridin sowie 1,5-Diazabicyclo[4.3.0]non-5-en (DBN), 1,8-Diazabicyclo[5.4.0]undec-7-en (DBU), 1,1,3,3-Tetramethylguanidin (TMG), 7-Methyl-1,5,7-triazabicyclo[4.4.0]dec-5-en (MTBD) und 2,8,9-Triisopropyl-2,5,8,9-tetraaza-1--phosphabicyclo[3.3.3]undecan (TTPU). Weitere Beispiele geeigneter Basen sind Arylanionen und Cyclopentadienylanionen. Besonders bevorzugt ist die Verwendung von Erdalkali- oder Alkalimetallhydroxide, sowie sterisch gehinderter -alkoholate wie z.B. Kalium-tert.-butylat.

Bei der technischen Produktion von Verbindungen der Formel (I) stellen Polymerisationsprodukte von Verbindungen der Formel (III) unerwünschte Nebenprodukte dar, die weitestgehend vermieden werden sollen. Diese Polymerisationsprodukte entstehen beispielsweise durch Autokondensation der Verbindung der Formel (III) im Vorratsgefäß oder in der Vorlage bei hohen Temperaturen und führen zu reduzierten Reinheiten und Ausbeuten und können zudem Rohrzuleitungen verstopfen. Daher ist eine besondere Verfahrensführung vorteilhaft.

Das Verfahren wird daher vorzugsweise so durchgeführt, das beispielsweise zunächst die Verbindung der Formel (II), gegebenenfalls in einem oder mehreren Lösungsmitteln und gegebenenfalls in Gegenwart einer oder mehrerer geeigneter Basen vorgelegt und dann die Verbindung der Formel (III) zugesetzt wird. Alternativ kann die Verbindung der Formel (IIa), gegebenenfalls in einem oder mehreren Lösungsmitteln vorgelegt werden und in Gegenwart einer oder mehrerer geeigneter Basen mit der Verbindung der Formel (III) in Kontakt gebracht werden. Alternativ kann die Verbindung der Formel (IIa) oder der Formel (II), gegebenenfalls in einem oder mehreren Lösungsmitteln vorgelegt werden, mit einem Überschuss an Base versetzt werden und mit der Verbindung der Formel (IIIa) in Kontakt gebracht werden. Alternativ kann die Reaktion auch mit Verbindungen der Formel (IIIa) und Base durchgeführt werden und zwar derart, dass beispielsweise zunächst aus Verbindungen der Formel (IIIa) durch Zugabe geeigneter Basen die Verbindungen der Formel (III) hergestellt werden.

In einer weiteren Ausführungsform kann die Deprotonierung der Formel (IIIa) auch in situ erfolgen.

Als Lösungsmittel zur Durchführung des erfindungsgemäßen Verfahrens eignen sich insbesondere organische Lösungsmittel. Geeignete organische Lösungsmittel sind beispielsweise aliphatische, alizyklische oder aromatische, gegebenenfalls halogenierte Kohlenwasserstoffe, wie beispielsweise Benzol, Toluol, Xylol, verschiedene Petrolether, Hexan, Cyclohexan, Tetrachlorkohlenstoff; Ether, wie Diethylether, Methyltert.-butylether, Diisopropylether, Dioxan, Tetrahydrofuran oder Ethylenglykoldimethyl- oder -diethylether; Ketone, wie Aceton, 2-Butanon oder Methyl-isobutyl-keton; Amide, wie N,N-Dimethylformamid, N,N-Dimethylacetamid, N-Methyl-formanilid, N-Methylpyrrolidon, N-Methylcaprolactam oder Hexamethylphosphorsäuretriamid, Sulfoxide, wie Dimethylsulfoxid, Sulfone wie Tetramethylensulfon, oder Gemische solcher organischen Lösungsmittel. Bevorzugt sind aromatische Kohlenwasserstoffe, aliphatische und alizyklische Amide, Sulfoxide und Sulfolane, besonders bevorzugt sind *N,N*-Dimethylacetamid, *N,N*-Dimethylformamid, p-Xylol und Xylol Isomerengemisch oder Mischungen.

Die Reaktionstemperatur kann beispielsweise zwischen 20 °C und 300 °C liegen. Bevorzugt liegt die Reaktionstemperatur zwischen 125 °C und 200 °C, besonders bevorzugt zwischen 135°C und 180 °C.

Beispielsweise kann die Temperatur der Verbindung der Formel (III) im Vorratsgefäß zwischen - 30 °C und 20 °C liegen. Damit wird die Autokondensation im Vorratsgefäß im Wesentlichen verhindert.

Das erfindungsgemäße Verfahren wird vorzugsweise im Wesentlichen frei von Übergangsmetallen der Gruppen 4 bis 12 des Periodensystems der Elemente, wie insbesondere Kupfer durchgeführt. Der Begriff "im Wesentlichen frei" bedeutet einen Gehalt an Übergangsmetall bezogen auf die Summe der Masse der zu koppelnden Verbindungen der Formel (II) und der Formel (III) von 0 bis 1000 ppm, bevorzugt 0 - 10 ppm.

Prinzipiell kann bei variablem Druck gearbeitet werden. Vorzugsweise wird bei Umgebungsdruck gearbeitet.

Bevorzugt wird die Zugabe der Verbindung der Formel (III) oder der Formel (IIIa) so durchgeführt, dass die Verhältnisse der Stoffmengen der Verbindung der Formel (II) und der Verbindung der Formel (III) oder der Formel (IIIa) bei der Zugabe zwischen 5:1 und 1000:1 liegen. Die Zugabe kann beispielsweise portionsweise, semikontinuierlich oder kontinuierlich erfolgen. Besonders bevorzugt erfolgt die Zugabe portionsweise. Besonders bevorzugt liegt das Verhältnis der Stoffmengen der Verbindung der Formel (II) zu der Verbindung der Formel (III) oder der Formel (IIIa) bei der Zugabe zwischen 5:1 und 20:1.

Beispielsweise kann das Stoffmengenverhältnis der eingesetzten Verbindungen der Formel (II) und der Verbindungen der Formel (III) oder der Formel (IIIa) bezogen auf die Gesamtreaktion zwischen 1:2 und 10: 1, bevorzugt zwischen 1:1 und 5:1, besonders bevorzugt zwischen 1:1 und 2:1 liegen.

In einer besonders bevorzugten Ausführungsform wird die Verbindung der Formel (IIa) mit der geeigneten Base in Kontakt gebracht. Die Vorlage wird dann auf Reaktionstemperatur erwärmt. Dann wird die Verbindung der Formel (IIIa) portionsweise so zugegeben, dass das Stoffmengenverhältnis der Verbindung der Formel (II) zu der Verbindung der Formel (IIIa) bei der Zugabe zwischen 5:1 und 20:1 liegt. In einer bevorzugten Ausführungsform liegt die Reaktionstemperatur zwischen 135°C bis 180 °C.

Auf erfindungsgemäße Weise können die Verbindungen der Formel (I) in hohen Ausbeuten in technischen Prozessen hergestellt werden. Die Aufarbeitung kann in an sich bekannter Weise, z.B. durch Extraktion mit bekannten Lösungsmitteln wie beispielsweise Wasser und Methyl-tert.-butylether erfolgen.

Die erfindungsgemäß hergestellten Verbindungen der Formel (I) eignen sich insbesondere als Zwischenprodukte z.B. zur Herstellung von Feinchemikalien, Arzneimitteln, wie z.B. Antidepressiva, Antibiotika oder Serotoninwiederaufnahmehemmern und Agrochemikalien.

## Beispiele

### 1 . Darstellung von 4-[4-Trifluormethoxyphenoxy] pyridin

36,4 g Kalium-*tert*-butylat (0,32 mol) wurden bei Raumtemperatur unter Inertatmosphäre in 200 ml N,N-Dimethylacetamid gelöst. Anschließend wurden 35,3 g 4-Trifluormethoxyphenol (0,19 mol) innerhalb von 40 Minuten zur gerührten Lösung gegeben. Anschließend wurde die Lösung auf 100 °C erwärmt und 20 g 4-Chlorpyridin Hydrochlorid (0,13 mol) in 4 Portionen innerhalb von 2 h zur Reaktionslösung gegeben. Die Reaktionslösung wurde auf 140 °C erwärmt und für 24 h bei 140 °C gerührt. 5,0 g Kalium-tert-butylat (0,045 mol) wurden zugegeben und die Reaktion für weitere 16 h bei Rückfluss gerührt. Die Reaktionslösung wurde auf Raumtemperatur abgekühlt und mit 100 ml Wasser und 100 ml Methyltert.-butylether versetzt und mit 10%iger Salzsäure auf pH 1 bis 2 gestellt. Die Phasen wurden getrennt. Die wässrige Phase wurde mit 50 ml 15%iger Natronlauge auf pH 11 gestellt und 3 mal mit jeweils 100 ml Methyltert.-butylether extrahiert. Die vereinigten organischen Phasen wurden mit 500 ml Wasser gewaschen. Das Lösungsmittel wurde unter vermindertem Druck entfernt. Man erhielt 25,3 g 4-[4-Trifluormethoxyphenoxy]pyridin mit einer Reinheit von 85,5 Gew% (0,085 mol) und einer Ausbeute von 65,5 Mol% d. Theorie.

## 2. Darstellung von 4-[4-Methylphenoxy]pyridin

40,4 g Kalium-tert-butylat (0,36 mol) wurden bei Raumtemperatur unter Inertatmosphäre in 220 g N,N-Dimethylacetamid gelöst. Anschließend wurden 19,5 g (0,18 mol) p-Kresol innerhalb von 40 Minuten zur gerührten Lösung gegeben. Anschließend wurde die Lösung auf 140 °C erwärmt und 18 g 4-Chlorpyridin Hydrochlorid (0,12 mol) in 10 Portionen im Abstand von 30 Minuten zur Reaktionslösung gegeben. Die Reaktionslösung wurde für 60 h bei 140 °C gerührt. Die Reaktionslösung wurde auf Raumtemperatur abgekühlt und mit 100 ml Wasser und 150 ml Methyl-tert.-butylether versetzt und mit 37%iger Salzsäure auf pH 1 gestellt. Die Phasen wurden getrennt. Die wässrige Phase wurde mit 50%iger Natronlauge auf pH > 11 gestellt und 2 mal mit jeweils 150 ml Methyl-tert.-butylether extrahiert. Die organischen Phasen wurden vereinigt. Das Lösungsmittel wurde unter vermindertem Druck entfernt. Man erhielt 20,5 g 4-[4-Methylphenoxy]pyridin (0,11 mol, 91 Mol% d. Theorie).

## 3. Darstellung von 4-Phenoxypyridin

75 g Kalium-tert-butylat (0,63 mol) wurden bei Raumtemperatur unter Inertatmosphäre in 375 g N,N-Dimethylacetamid gelöst. Anschließend wurden 30 g (0,32 mol) Phenol innerhalb von 40 Minuten zur gerührten Lösung gegeben. Anschließend wurde die Lösung auf 140 °C erwärmt und 32 g 4-Chlorpyridin Hydrochlorid (0,21 mol) innerhalb von 5 h Portionsweise zur Reaktionslösung gegeben. Die Reaktionslösung wurde für 19 h bei 140 °C gerührt. Die Reaktionslösung wurde auf Raumtemperatur abgekühlt und mit 100 ml Wasser und 150 ml Methyl-tert.-butylether versetzt und mit 37%iger Salzsäure auf pH 1 gestellt. Die Phasen wurden getrennt. Die wässrige Phase wurde mit 50%iger Natronlauge auf pH >11 gestellt und 2 mal mit jeweils 150 ml Methyl-tert.-butylether extrahiert. Die organischen Phasen wurden vereinigt. Das Lösungsmittel wurde unter vermindertem Druck entfernt. Man erhielt 34 g Phenoxypyridin (0,19 mol, 94 Mol% d. Theorie).

## 4. Darstellung von 4-[4-Methoxyphenoxy]pyridin

58,0 g Kalium-tert-butylat (0,52 mol) wurden bei Raumtemperatur unter Inertatmosphäre in 232,3 g N,N-Dimethylacetamid gelöst. Anschließend wurden 48,2 g (0,39 mol) 4-Methoxylphenol zur gerührten Lösung gegeben. Anschließend wurde die Lösung auf 136-140 °C erwärmt und 40 g Chlorpyridin Hydrochlorid (0,26 mol) in 8 Portionen innerhalb von 18 h zudosiert. Die Reaktionslösung wurde anschließend für 7 h bei 140 °C gerührt. Die Reaktionslösung wurde auf Raumtemperatur abgekühlt und mit 325 g Wasser und 300 g Methyl-tert.-butylether versetzt und mit 37%iger Salzsäure auf pH 1 gestellt. Die Phasen wurden getrennt. Die wässrige Phase wurde mit 50%iger Natronlauge auf pH > 11 gestellt und 2 mal mit jeweils 200 g Methyl-tert.-butylether extrahiert. Die organischen Phasen wurden vereinigt. Das Lösungsmittel wurde unter vermindertem Druck entfernt. Man erhielt 45,5 g 4-[4-Methoxyphenoxy]pyridin (0,23 mol, 87 Mol% d. Theorie ).

## 5. Darstellung von 4-[2-Trifluormethoxyphenoxy]pyridin

16,7 g Kalium-tert-butylat (0,15 mol) wurden bei Raumtemperatur unter Inertatmosphäre in 67 g N,N-Dimethylacetamid gelöst. Anschließend wurden 19,9 g (0,11 mol) 2-Trifluormethoxyphenol zur gerührten Lösung gegeben. Anschließend wurde die Lösung auf 140 °C erwärmt und 11,2 g Chlorpyridin Hydrochlorid (0,075 mol) in 10 Portionen im Abstand von 30 Minuten in die Reaktionsmischung gegeben und für 60 h bei 140 °C gerührt. Die Reaktionslösung wurde auf Raumtemperatur abgekühlt und mit 150 ml Wasser und 150 ml Methyl-tert.-butylether versetzt und mit 37%iger Salzsäure auf pH 1 gestellt. Die Phasen wurden getrennt. Die wässrige Phase wurde mit 50%iger Natronlauge auf pH > 11 gestellt und mit 2 mal jeweils 150 ml Methyl-tert.-butylether extrahiert. Die organischen Phasen wurden vereinigt. Das Lösungsmittel wurde unter vermindertem Druck entfernt. Man erhielt 14,2 g 4-[2-Trifluormethoxyphenoxy]pyridin (0,057 mol, 75 Mol% d. Theorie).

## Patentansprüche

1. Verfahren zur Herstellung von Verbindungen der Formel (I)

$$ARYL\text{-}O\text{-}HETEROARYL \qquad (I)$$

wobei ARYL für $C_6$-$C_{20}$-Aryl steht, das gegebenenfalls einfach oder mehrfach mit Resten substituiert ist, die unabhängig voneinander ausgewählt sind aus der Gruppe

Alkyl, Alkenyl, Alkoxy, Alkoxycarbonyl, Alkoxycarbonylamino, Dialkylamino, Aryl, Arylalkyl, Halogen, Halogenalkyl, Halogenalkylen, Halogenalkoxy, Halogenalkylthio, 5- bis 6-gliedriges Heteroaryl, und 3- bis 7-gliedriger gesättigter oder teilweise ungesättigter Heterozyklus

und

HETEROARYL für Pyrazinyl, Pyridyl, Pyrimidinyl oder Pyridazinyl steht, das gegebenenfalls einfach oder mehrfach mit Resten substituiert ist, die unabhängig voneinander ausgewählt sind aus der Gruppe

Alkyl, Alkenyl, Alkoxy, Alkoxycarbonyl, Alkoxycarbonylamino, Dialkylamino, Aryl, Arylalkyl, Halogen, Halogenalkyl, Halogenalkylen, Halogenalkoxy, Halogenalkylthio, 5- bis 6-gliedriges Heteroaryl, 3- bis 7-gliedriger gesättigter oder teilweise ungesättigter Heterozyklus
das **dadurch gekennzeichnet ist, dass** Verbindungen der Formel (II)

$$\text{ARYL-O}^{-} \text{ Kat}^{+} \qquad \text{(II)}$$

wobei ARYL die vorgenannte Bedeutung besitzt und Kat$^{+}$ ein beliebiges einfach geladenes Kation oder ein 1/n-tes Äquivalent eines n-wertigen Kations darstellt,
mit einer Verbindung der Formel (III) umgesetzt wird

$$\text{HETEROARYL-Y} \qquad \text{(III)}$$

wobei HETEROARYL die vorgenannte Bedeutung besitzt und Y für ein Halogen oder Pseudohalogen steht.

2. Verfahren nach Anspruch 1, wobei ARYL für $C_6$-$C_{20}$-Aryl steht, das gegebenenfalls einfach oder mehrfach substituiert ist mit Resten die unabhängig voneinander ausgewählt sind aus der Gruppe Alkyl, Aryl, Alkoxy, Halogenalkyl ,Halogenalkoxy und Halogenalkylthio.

3. Verfahren nach Anspruch 1 oder 2, **dadurch gekennzeichnet, dass** ARYL für $C_6$-$C_{20}$-Aryl steht, das gegebenenfalls einfach oder mehrfach substituiert ist mit Resten, die unabhängig voneinander ausgewählt sind aus der Gruppe Trifluormethoxy, Methoxy und Methyl.

4. Verfahren nach einem oder mehreren der Ansprüche 1 bis 3, **dadurch gekennzeichnet, dass** HETEROARYL für Pyridyl steht, das gegebenenfalls einfach oder mehrfach substituiert ist mit Resten, die unabhängig voneinander ausgewählt sind aus der Gruppe Alkyl, Aryl, Alkoxy, Halogenalkyl, Halogenalkoxy und Halogenalkylthio.

5. Verfahren nach einem oder mehreren der Ansprüche 1 bis 4, **dadurch gekennzeichnet, dass** HETEROARYL für 4-Pyridyl steht, das gegebenenfalls einfach oder mehrfach substituiert ist mit Resten, die unabhängig voneinander ausgewählt sind aus der Gruppe Trifluormethoxy, Methoxy und Methyl.

6. Verfahren nach einem oder mehreren der Ansprüche 1 bis 5, **dadurch gekennzeichnet, dass** HETEROARYL-Y für 4-Chlorpyridin steht.

7. Verfahren nach einem oder mehreren der Ansprüche 1 bis 6, **dadurch gekennzeichnet, dass** Verbindung der Formel (I) für 4-[4-Trifluormethoxyphenoxy]pyridin, 4-[4-Methylphenoxy]pyridin, 4-Phenoxypyridin, 4-[4-Methoxyphenoxy]pyridin und 4-[2-Trifluormethoxyphenoxy]pyridin steht.

8. Verfahren nach einem oder mehreren der Ansprüche 1 bis 7, **dadurch gekennzeichnet, dass** die Zugabe der Verbindung der Formel (III) oder der Formel (IIIa) zu der Verbindung der Formel (II) derart erfolgt, dass das Verhältnis der Stoffmenge von Verbindung der Formel (II) zu Verbindungen der Formel (III) oder der Formel (IIIa) bei der Zugabe zwischen 5:1 und 1000:1 liegt.

9. Verfahren nach Anspruch 8, **dadurch gekennzeichnet, dass** die Zugabe derart erfolgt, dass das Verhältnis der Stoffmenge von Verbindungen der Formel (II) zu Verbindungen der Formel (III) oder der Formel (IIIa) bei der Zugabe zwischen 5:1 und 20:1 liegt.

10. Verfahren nach einem oder mehreren der Ansprüche 1 bis 9, **dadurch gekennzeichnet, dass** die Reaktionstemperatur zwischen 20 °C und 300 °C liegt.

11. Verfahren nach Anspruch 10, **dadurch gekennzeichnet, dass** die Reaktionstemperatur zwischen 135 °C und 180 °C liegt.

12. Verfahren nach einem oder mehreren Ansprüchen 1 bis 11, **dadurch gekennzeichnet, dass** die Reaktion im Wesentlichen frei von Übergangsmetallen der Gruppen 4 bis 12 des Periodensystems der Elemente durchgeführt wird.

13. Verfahren nach einem oder mehreren der Ansprüche 1 bis 12, **dadurch gekennzeichnet, dass** die Reaktion in Gegenwart von N,N-Dimethylacetamid, N,N-Dimethylformamid, p-Xylol oder Xylol Isomerengemisch stattfindet.

14. Verfahren nach einem oder mehreren Ansprüchen 1 bis 13, **dadurch gekennzeichnet, dass** die Herstellung der Verbindung (II) aus der Verbindung (IIa) unter Verwendung geeigneter Basen deren korrespondierende Säuren einen pKa-Wert > 10 aufweisen, durchgeführt wird.

15. Verwendung der Verbindung (I), die nach einem oder mehreren der Ansprüche 1 bis 14 hergestellt wird, für die Herstellung von Arzneimitteln, Agroche-

mikalien oder Zwischenprodukten.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- JP 2001002644 A
- DE 69829048 T2
- DE 60201819 T2

**In der Beschreibung aufgeführte Nicht-Patentliteratur**

- *Chem. Ber.,* 1956, vol. 89, 2921-2933
- **Chemg et al.** *Tetrahedron,* 2002, vol. 58, 4931-4935
- **Angelo et al.** *Tetrahedron Letters,* 2006, vol. 47, 5045-5048